# EUROPEAN PATENT APPLICATION

(11) **EP 4 134 105 A1**
(43) Date of publication of application: **15.02.2023**
(21) Application number: 21768139.4
(22) Date of filing: 15.03.2021
(51) Int. Cl.: A61K 48/00, A61K 9/127, A61K 9/00, A61P 11/00, C12N 5/0775

(54) **ATOMIZED INHALATION FORMULATION CONTAINING HUMAN CELL-DERIVED EXTRACELLULAR VESICLES, PREPARATION METHOD AND USE THEREOF**

(30) Priority: 13.03.2020 CN 202010177541
(71) Applicant: Cellular Biomedicine Group (Shanghai) Ltd., Xuhui District, Shanghai 200233 (CN); Cellular Biomedicine Group (Wuxi) Ltd., Wuxi, Jiangsu 214174 (CN)
(72) Inventor: DAI, Chengxiang, Shanghai 200233 (CN); LI, Ping, Shanghai 200233 (CN); WANG, Jing, Shanghai 200233 (CN); LI, Suke, Shanghai 200233 (CN); LEI, Jigang, Shanghai 200233 (CN); CHEN, Yinglu, Shanghai 200233 (CN); SONG, Xiaole, Shanghai 200233 (CN)
(74) Representative: Cabinet Becker et Associés
(86) International application number: PCT/CN2021/080869
(87) International publication number: WO 2021/180237

(57) **Abstract**

A pharmaceutical composition for treating diseases related to inflammation or injury such as ARDS, comprising: (a) an active material derived from human somatic cells, the active ingredient being an extracellular vesicle produced by human somatic cells; and (b) a pharmaceutically acceptable carrier. The somatic cells are selected from human tissue, bone marrow and/or blood-derived stem cells, etc. The dosage form of the pharmaceutical composition is selected from an atomized inhalation agent, eye drops and nasal drops. The pharmaceutical composition has storage stability and high dispersibility superior to those of a living cell formulation. A method for preparing an extracellular vesicle, comprising steps of culturing human somatic cells, removing cells from a culture system, mixing a culture solution with polyethylene glycol to form an extracellular vesicle modified by PEG, centrifuging, resuspending, etc. The formulation of the extracellular vesicle can be used for preparing a medicament for preventing and/or treating inflammations or injuries.

## Description

### Technical field

The present invention relates to the field of biomedicine, and more particularly to an atomized inhalation formulation containing human cell-derived extracellular vesicles, and preparation method and use thereof.

### Background technology

Acute respiratory distress syndrome (ARDS) is a common cause of respiratory failure in critically ill patients and is defined as acute exacerbations of noncardiogenic pulmonary edema and hypoxemia requiring mechanical ventilation.

ARDS is most commonly associated with pneumonia, sepsis, inhalation of gastric contents or severe trauma, and is found in approximately 10% of patients in intensive care units worldwide. Despite some progress made in the past few decades, the mortality rate is still as high as 30-40% in most studies. There is a total of approximately 200,000 cases of ARDS each year in the United States, and the hospital mortality rate is as high as 38.5%, which has not improved significantly over past decades.

Acute lung injury (ALI) and ARDS share the same pathological changes, the most common change being diffused alveolar damages. The pathological basis of ALI/ARDS is the damages of alveolar epithelium and alveolar capillary endothelium caused by local inflammation and uncontrolled inflammation in the lung mediated by a variety of inflammatory cells (macrophages, neutrophils, lymphocytes, etc.). Its main pathological feature is the formation of protein-rich pulmonary edema and hyaline membrane in alveolar exudate caused by increased pulmonary microvascular permeability, which may be accompanied by pulmonary interstitial fibrosis. The pathophysiological changes are mainly decreased lung compliance, increased intrapulmonary shunt and imbalance of ventilation to blood flow ratio. The clinical manifestations are respiratory rate and respiratory distress, refractory hypoxemia, diffuse infiltration of both lungs shown by chest X-ray, and failures of multiple organs as complications in later stages.

Clinical diagnosis criteria based on expert consensus are used for the diagnosis of ARDS. The focus of patient management is to implement lung-protection ventilation strategies. No specific drug therapy has yet been identified. Long-term prognosis of surviving ARDS patients is increasingly recognized as an important research goal, as many ARDS patients survive with persistent sequelae in organ functions and/or the cognitive and mental state. Future research directions include promoting early identification of ARDS, adding prognostic and/or predictive functions in clinical studies to identify subgroups that may benefit from treatments, and continuing the efforts to understand the underlying mechanisms of lung injuries.

Existing treatments for acute respiratory distress syndrome aim at modulating inflammation or inflammatory cascades and have been used in the studies of ARDS. However, existing anti-inflammatory drugs such as corticosteroids, neutrophil elastase inhibitors, granulocyte colony-stimulating factors, statins and ω-3 fatty acids, as well as surfactants aiming at improving lung respiratory mechanics such as inhaled β receptor agonists, nitric oxide, etc., have not shown a benefit in reducing mortality. Only supportive therapies that reduce pressure lung injury (barotrauma) during mechanical ventilation, such as lung-protective ventilation with neuromuscular blockers or prone ventilation, have shown an improvement in mortality, and therefore these therapies remain the main means of ARDS treatment. So far, existing treatment methods for ARDS can neither reduce the mortality rate, nor reduce pulmonary fibrosis and other organ damages or neuropsychiatric sequelae in survivors.

From the end of 2019 to 2020, the new coronavirus (SARS-CoV-2) infectious pneumonia broke out in China and around the world, causing severe pneumonia, acute respiratory distress syndrome (ARDS), etc. For severe pneumonia and ARDS caused by coronaviruses such as SARS-CoV-2, there is currently no effective vaccine or antiviral drug. These infectious diseases have seriously affected people's lives and health, and it is imminent to develop effective therapeutic drugs. It is of great social significance to develop low-toxicity and high-efficacy drugs for diseases of pneumonia and ARDS caused by coronaviruses such as SARS-CoV-2 to meet the clinical needs of patients at home and abroad.

In view of the above, there is an urgent need in this field to develop drugs with high efficacy, low toxicity, high safety and feasibility for mass production for the treatment of ARDS and other diseases.

### Summary of the invention

The purpose of the present invention is to provide a drug with high efficacy, low toxicity, high safety and feasibility for mass production for the treatment of ARDS and other diseases.

In a first aspect of the present invention, a pharmaceutical composition is provided, comprising (a) an active ingredient derived from human somatic cells, which is an extracellular vesicle produced by human somatic cells; and (b) a pharmaceutically acceptable carrier.

In another preferred example, the pharmaceutical composition is an injection or a cell-free pharmaceutical composition.

In another preferred example, "cell-free" means that the pharmaceutical composition does not contain living or dead cells.

In another preferred example, the somatic cells are selected from: stem cells, progenitor cells or immune cells derived from human tissues, bone marrow and/or blood, or a combination thereof.

In another preferred example, the dosage form of the pharmaceutical composition is selected from: liquid dosage form, and solid dosage form (such as lyophilized dosage form).

In another preferred example, the dosage form of the pharmaceutical composition is selected from: an atomized inhalation preparation, eye drops, or nasal drops.

In another preferred example, the pharmaceutical composition has the following characteristics:
(P1) Better storage stability than living cell formulations; preferably, the storage is at room temperature and/or low temperature (for example, -196°C to 25°C, preferably -100°C to 0°C); "better storage stability than living cell formulations" means that the stability of the pharmaceutical composition is better than that of a formulation containing living cells under the same conditions; and
(P2) High dispersibility; preferably, when the pharmaceutical composition is a liquid formulation containing water (for example, a solution prepared with saline) and placed at 0-25°C for 6-24 hours, it is colorless and transparent without visible floccules or precipitation.

In another preferred example, the pharmaceutical composition is administered to a site other than the skin.

In another preferred example, the pharmaceutical composition is administered to a site selected from: the bulbar conjunctiva, palpebral conjunctiva, retina, oral cavity, nasal cavity, upper respiratory tract, lower respiratory tract, gastrointestinal tract, lung, or a combination thereof.

In another preferred example, the pharmaceutical composition is administered to cells selected from: vascular endothelial cells, type 1 alveolar epithelial cells, type 2 alveolar epithelial cells, mononuclear macrophages, neutrophils, dendritic cells, antigen presenting cells, T lymphocytes, fibroblasts, central nerve cells, peripheral nerve cells and the fibers of their ending, or a combination thereof.

In another preferred example, the stem (progenitor) cells are selected from: mesenchymal stem cells derived from human adipose tissue, alveolar epithelial progenitor cells derived from human lung tissue, mesenchymal stem cells derived from Wharton's jelly of human umbilical cord, mesenchymal stem cells derived from human endometrial tissue, stem cells derived from human placental tissue, epithelial cells derived from human placental amniotic membrane, mononuclear cells in human blood, hematopoietic stem cells in human bone marrow tissue, mesenchymal stem cells in human bone marrow tissue, mesenchymal stem cells derived from human periosteum, keratinocytes derived from human skin tissue, dendritic cells derived from human blood tissue, CD4-positive T lymphocytes derived from human blood tissue, platelets derived from human blood tissue, or a combination thereof.

In another preferred example, the extracellular vesicles comprise a nanovesicle enveloped in a lipid bilayer membrane structure.

In another preferred example, the diameter of the nanovesicle ranges between 30-1,000 nanometers.

In another preferred example, the nanovesicle contains different types of microribonucleic acids (miRNAs), small nuclear ribonucleic acid (sRNAs), non-coding DNA fragments, transfer ribonucleic acids (t-RNAs), soluble cytokines, growth factors, and other proteins of specific functions.

In another preferred example, the soluble cytokines are selected from: soluble and free lipoprotein molecules, glycoprotein molecules, biosignaling molecules, transforming growth factor β (TGF β), hepatocyte growth factor (HGF), vascular endothelial growth factor (VEGF), keratinocyte growth factor (KGF), interleukin 10 (IL-10), interleukin 1β receptor antagonist (IL-1βA), or a combination thereof.

In another preferred example, the soluble cytokines are soluble cytokines produced by human somatic cells.

In another preferred example, the soluble cytokines comprise cytokines produced paracrinely from human somatic cells cultured in a natural state, cytokines expressed from human somatic cells modified with exogenous genes, or a combination thereof.

In another preferred example, the extracellular vesicle specifically expresses the following proteins: CD9, CD63, CD81 and TSG101; it does not express or essentially does not express protein CANX.

In another preferred example, the diameter of the extracellular vesicle is preferably 50-500 nm.

In another preferred example, the diameter is an average diameter.

In another preferred example, the cells used to produce the extracellular vesicle comprise cells from the following sources:
(a) Cells obtained by direct separation from human tissues and purification;
(b) Cells obtained by minimal manipulation in a GMP laboratory to proliferate the cells after direct separation from human tissues and purification;
(c) Cells obtained by specific gene modification, specific gene editing, specific gene transduction, specific miRNA introduction in a GMP laboratory; and
(d) Cells obtained by pretreatment under special culture conditions in a GMP laboratory.

In another preferred example, the cells used to produce the extracellular vesicle comprise primary cells and passaged cells with a passage number of 1-10.

In another preferred example, the cells used to produce the extracellular vesicle comprise cells that are not genetically manipulated and cells that have been genetically manipulated.

In another preferred example, the genetic manipulation comprises gene editing, gene introduction, gene knock-down, gene knock-out, or a combination thereof.

In another preferred example, the cells used to produce the extracellular vesicle comprise pretreated cells.

In another preferred example, the somatic cells are adipose-derived mesenchymal stem (progenitor) cells, placental amniotic membrane-derived mesenchymal stem cells, or a combination thereof.

In another preferred example, the adipose-derived mesenchymal stem (progenitor) cells are obtained by the following means: taking fat by abdominal liposuction or abdominal dermolipectomy from a healthy male or female volunteer who has passed ethical review, has been registered, has signed the informed consent, and has been strictly examined by a laboratory to meet the criteria for an adipose stem (progenitor) cell bank, placing the fat in a cell storage solution, transporting it at a low temperature to a qualified GMP laboratory, and obtaining adipose mesenchymal progenitor cells, i.e., working bank cells (intermediate product), by separation, purification and proliferation.

In another preferred example, the mesenchymal stem (progenitor) cells derived from human adipose tissue are P1-P6 adipose mesenchymal stem (progenitor) cells produced in accordance with a production process for intermediate products in a GMP production laboratory, tested negative for various pathogens, with a percentage of approximately 98% of specific surface markers such as CD73-positive, CD90-positive and CD105-positive cells and a percentage smaller than 2% of CD34-positive/CD45-positive and HLA-DR-positive cells, and meeting the detection criteria for antibiotic residues and serum and serum substitute residues.

In another preferred example, the adipose mesenchymal stem (progenitor) cells are P3-P4 adipose mesenchymal stem (progenitor) cells.

In another preferred example, the somatic cells are adipose tissue-derived mesenchymal stem (progenitor) cells stored under a deep hypothermic condition at -196°C to -80°C (preferably, -196°C to -135°C) for 0-36 months (preferably 0-24 months or 0.5-24 months).

In another preferred example, the production process for the human somatic cell-derived extracellular vesicle and the soluble cytokines comprises the following steps:
(1) Seeding the human somatic cells in α MEM basal culture medium containing 5% EliteGro, culturing them in a HyperFlask culture flask under culture conditions of 37 ± 1°C and 5 ± 0.5% CO₂ until the cell confluency reaches 80 ± 10%, then using a specific pretreated medium for culturing under culture conditions of 37 ± 1°C and 5 ± 0.5% CO₂ for 36-72 hours; and
(2) Collecting the cell culture supernatant, and performing separation by differential centrifugation combined with polyethylene glycol (PEG) precipitation.

In another preferred example, the differential centrifugation in combination with PEG precipitation comprises the following steps: filtering (by using, for example, a 0.22 µm filter) by using PEG of PEG3000-PEG9000, PEG with PBS being configured to 8%-30% to remove bacteria, adding it to a treated conditioned medium at the certain ratio (for example, volume ratio of 1:1), placing it at 4°C overnight for incubation, performing centrifugal separation under 3,000-5,000 g at 4°C for 30-60 minutes, removing the supernatant, adding pre-cooled PBS to resuspend the precipitate, performing ultracentrifugation under 100,000-120,000 g at 4°C for 60-120 minutes, removing the supernatant, adding an appropriate amount of isotonic sodium chloride solution, and resuspending the precipitate to obtain the human somatic cell-derived extracellular vesicle and the soluble cytokines.

In another preferred example, the extracellular vesicle obtained by the production process has the following biomarker features: CD9 positive (i.e., CD9⁺), CD63 positive, CD81 positive, TSG101 positive, and CANX negative.

In another preferred example, the production process has the following characteristic: a clinical grade production scale for cell products under GMP laboratory conditions.

In another preferred example, the clinical grade production scale means that 1 production operation unit can separate 800-1,200 ml of conditioned medium from 2 cell factories to obtain 2-5 × 10¹¹ extracellular vesicles, and that one batch produced from one production operation unit can meet the need of the local usage for 100-250 patients if a total of 2-5 × 10⁹ extracellular vesicles are used locally for each patient. Or, one batch produced from 100 production operation units can yield extracellular vesicles to be used for 10,000-25,000 people for 5-6 days.

In another preferred example, the production process is characterized in that: the separated extracellular vesicles and the soluble cytokines are cross-linked with PEG of a specific molecular weight to form hydrophilic particles of a specific size (for example, less than 3 microns) to deliver high stability when stored frozen at -196°C to -20°C and high dispersibility in an aqueous solution at room temperature.

In another preferred example, the extracellular vesicles, soluble cytokines and hydrophilic particles cross-linked by PEG are dispersed in an isotonic sodium chloride solution, a low molecular hyaluronic acid solution, artificial tears or a gel solution, thereby forming an atomized inhalation liquid, eye drops, nasal drops, or a topical gel preparation.

In another preferred example, the pharmaceutical composition is administered to mucosal cells, and the mucosal cells can take in extracellular vesicles.

In another preferred example, the pharmaceutical composition of the present invention is an atomized inhalation preparation.

In another preferred example, the pharmaceutical composition (especially the atomized inhalation preparation) of the present invention is used for the treatment of infectious lung injury, preferably viral lung injury, and more preferably lung injury caused by infection of coronaviruses (for example, the SARS-CoV virus and SARS-CoV-2 virus).

In another preferred example, the pharmaceutical composition is an atomized inhalation liquid prepared from extracellular vesicles and soluble cytokines derived from adipose tissue-derived progenitor cells and placental amniotic membrane-derived mesenchymal stem cells with an isotonic sodium chloride solution.

In another preferred example, the pharmaceutical composition is used to treat diseases caused by viruses.

In another preferred example, the viruses are selected from the group consisting of: influenza viruses, SARS coronavirus, SARS-Cov-2, and MERS coronavirus.

In another preferred example, the pharmaceutical composition (for example, atomized inhalation liquid) is used for the treatment of viral acute lung injury, and the treatment with atomized inhalation can prevent the release of acute lung injury inflammatory factors, reduce the infiltration of a high protein liquid in the alveoli and alveolar epithelium cell damage, significantly increase the success rate of rescue of patients with acute lung injury, and improve lung functions and the quality of life of surviving patients.

In a second aspect of the present invention, a method for preparing an extracellular vesicle is provided, comprising the following steps:
(S1) Culturing human somatic cells (for example, adipose mesenchymal stem (progenitor) cells) to a predetermined confluency (for example, 75-90%);
(S2) Under conditions suitable for EV production, continuing the culture of the cells for a period of time T1; T1 is usually 24-72 hours, preferably 30-60 hours;
(S3) Removing the cells from the culture system to separate and obtain a culture medium containing extracellular vesicles, i.e., "conditioned medium";
(S4) Mixing the conditioned medium with polyethylene glycol (PEG) to form a first mixture, and placing it for a period of time T2, thereby forming PEG-modified extracellular vesicles; T2 is usually 6-60 hours, preferably 12-48 hours;
(S5) Centrifuging the first mixture from the previous step to precipitate the PEG-modified extracellular vesicles, and removing the supernatant to obtain a PEG-modified extracellular vesicle precipitate;
(S6) Resuspending the PEG-modified extracellular vesicle precipitate obtained from the previous step to obtain a first resuspension mixture;
(S7) Centrifuging the first resuspension mixture to precipitate the PEG-modified extracellular vesicles, and removing the supernatant to obtain a PEG-modified extracellular vesicle precipitate;
(S8) Resuspending the PEG-modified extracellular vesicle precipitate obtained from the previous step to obtain a medically acceptable extracellular vesicle formulation (i.e., PEG-EVs-factors formulation);

In another preferred example, the method further comprises:
(S9) Mixing the medically acceptable extracellular vesicle formulation (or active ingredient) with a pharmaceutically acceptable carrier to form a pharmaceutical composition.

In another preferred example, the method further comprises making the pharmaceutical composition into an atomized inhalation preparation, an injection, or a lyophilized preparation.

In a third aspect of the present invention, an extracellular vesicle formulation is provided, which is prepared by the method described in the second aspect of the present invention.

In another preferred example, the extracellular vesicle formulation comprises a nanovesicle enveloped in a lipid bilayer membrane structure.

In another preferred example, the diameter of the nanovesicle ranges between 30-1,000 nanometers.

In another preferred example, the nanovesicle contains different types of microribonucleic acids (miRNAs), small nuclear ribonucleic acids (sRNAs), non-coding DNA fragments, transfer ribonucleic acids (t-RNAs), soluble cytokines, growth factors, and other proteins of specific functions.

In another preferred example, the soluble cytokines are selected from the group consisting of: soluble and free lipoprotein molecules, glycoprotein molecules, biosignaling molecules, transforming growth β (TGF β), hepatocyte growth factor (HGF), vascular endothelial growth factor (VEGF), keratinocyte growth factor (KGF), interleukin 10 (IL-10), interleukin 1β receptor antagonist (IL-1βA), or a combination thereof.

In another preferred example, the soluble cytokines are soluble cytokines produced by human somatic cells.

In another preferred example, the soluble cytokines comprise cytokines produced paracrinely from human somatic cells cultured in a natural state, cytokines expressed from human somatic cells modified with exogenous genes, or a combination thereof.

In another preferred example, the extracellular vesicle specifically expresses the following proteins: CD9, CD63, CD81 and TSG101; it does not express or essentially does not express protein CANX.

In a fourth aspect of the present invention, a use of the pharmaceutical composition described in the first aspect of the present invention or the extracellular vesicle formulation described in the third aspect is provided, and they are used to prepare a drug for preventing and/or treating inflammation or injury.

In another preferred example, the inflammation is selected from the group consisting of: viral infectious inflammation, bacterial infectious inflammation, fungal infectious inflammation, autoimmune response inflammation, or a combination thereof;

The injury is selected from the group consisting of: ischemic injury, hypoxic injury, chemical injury, physical injury, or a combination thereof.

In another preferred example, the drugs are used to treat diseases caused by viruses.

In another preferred example, the viruses are selected from the group consisting of: influenza viruses, SARS coronavirus, SARS-Cov-2, and MERS coronavirus.

In a fifth aspect of the present invention, a method for preventing and/or treating inflammation or injury is provided, comprising the following step: administering the pharmaceutical composition described in the first aspect of the present invention or the extracellular vesicle formulation described in the third aspect to a subject in need.

In another preferred example, the inflammation is selected from the group consisting of: viral infectious inflammation, bacterial infectious inflammation, fungal infectious inflammation, autoimmune response inflammation, or a combination thereof.

In another preferred example, the injury is selected from the group consisting of: ischemic injury, hypoxic injury, chemical injury, physical injury, or a combination thereof.

In another preferred example, the drug is used to treat acute respiratory distress syndrome. In another preferred example, the drug is an atomized inhalation liquid.

In another preferred example, the drug is used to treat viral acute lung injury.

In another preferred example, the drug is used for treatments through atomized inhalation to prevent the release of acute lung injury inflammatory factors and to reduce the infiltration of a high protein liquid in the alveoli and the alveolar epithelium cell damage.

In another preferred example, the subject is human.

It should be understood that, within the scope of the present invention, the above technical features of the present invention and the technical features described in detail below (e.g., the embodiments) may be combined to form a new or preferred technical solution. Due to the space limitation, it will not be detailed here.

### Description of the drawings

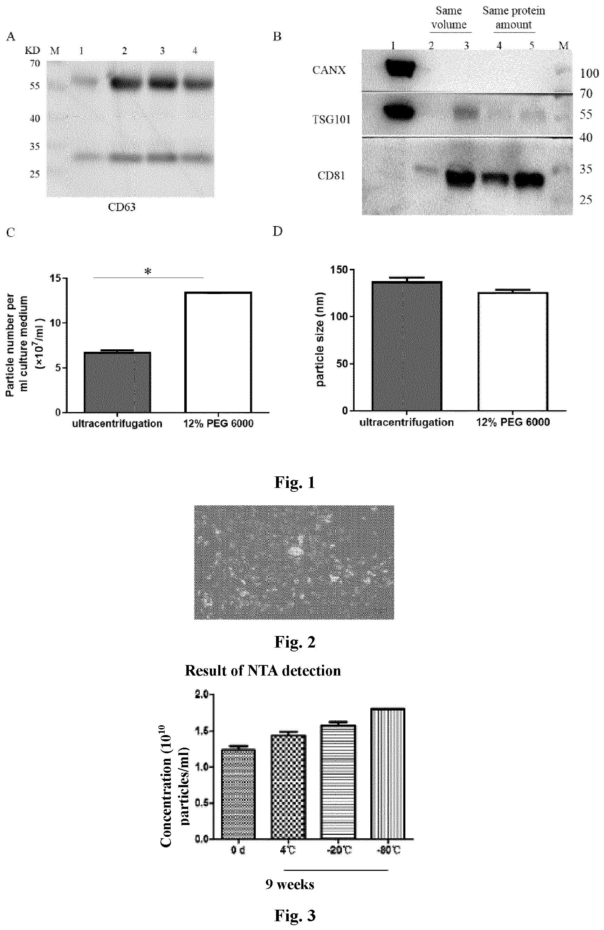

Figure 1 shows the yield of extracellular vesicles collected by PEG precipitation and ultracentrifugation detected by Western blot and NTA. In the figure, Figure (1A): M is the marker; 1-4 are respectively the CD63 expressions of extracellular vesicles separated by 8% PEG 6000, 12% PEG 6000, 16% PEG 6000 and 20% PEG 6000 (with the sample loaded with the same amount of proteins); Figure (1B): M is the marker; 1 is haMSCs; 2 and 4 are the protein expressions of extracellular vesicles separated by ultracentrifugation; 3 and 5 are the protein expressions of extracellular vesicles separated by 12% PEG 6000; Figure (1C): the concentrations of extracellular vesicle particles separated by PEG 6000 and ultracentrifugation detected by NTA; Figure (1D): diameters of extracellular vesicle particles separated by PEG 6000 and ultracentrifugation detected by NTA; * p < 0.05.
Figure 2 shows the intake of extracellular vesicles by fibroblasts. In the figure, blue: Hoechst stained cell nucleus; green: extracellular vesicle marked by PKH-67; scale: 100 µm.
Figure 3 shows the number of extracellular vesicles stored at 4°C, -20°C and -80°C for 9 weeks.
Figure 4 shows the expression of the specific marker CD81 of extracellular vesicles stored at 4°C, -20°C and -80°C for 9 weeks
Figure 5 shows the state of extracellular vesicles under a transmission electron microscope after thawing for half an hour at room temperature. In the figure, Figure 5A is the state of extracellular vesicles under a transmission electron microscope after storage at -80°C for 1 week, and Figure 5B is the state of extracellular vesicles under a transmission electron microscope after thawing for half an hour at room temperature.
Figure 6 shows the particle concentration and particle size of extracellular vesicles after thawing for half an hour at room temperature.

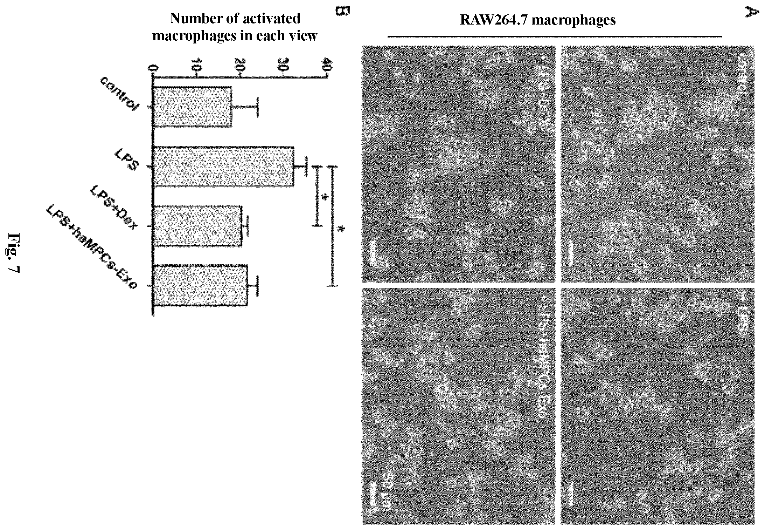

Figure 7 shows the inhibition of LPS-induced macrophage activation by extracellular vesicles of human-derived adipose mesenchymal progenitor cells. In the figure, (A) is morphological observation of pro-inflammatory macrophages; (B) is morphological statistical analysis of pro-inflammatory macrophages. Control is the control group, LPS is a macrophage group treated with lipopolysaccharide alone, LPS+Dex is a group treated with lipopolysaccharide and dexamethasone, LPS+haMPCs-Exo is a group treated with lipopolysaccharide and extracellular vesicles of adipose mesenchymal progenitor cells, the red arrows indicate activated macrophages (pro-inflammatory macrophages); ^{∗} p < 0.05, and the scale is 50 µm.

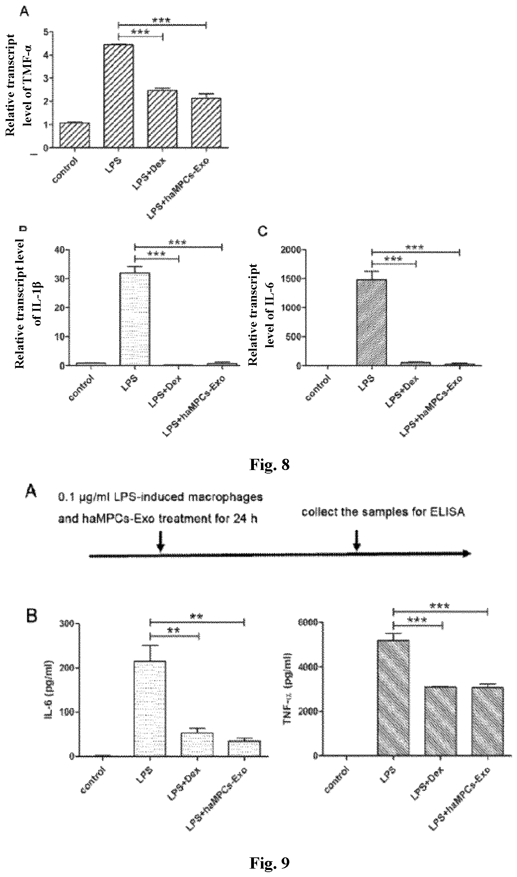

Figure 8 shows the inhibition of the gene expression of pro-inflammatory factors in LPS-induced macrophages by extracellular vesicles of human-derived adipose mesenchymal progenitor cells. In the figure, (A), (B) and (C) respectively show the gene expression levels of macrophage inflammatory factors TNF-α, IL-1β and IL-6 detected by real-time fluorescence quantitative PCR. Control is the control group, LPS is a macrophage group treated with lipopolysaccharide alone, LPS+Dex is a group treated with lipopolysaccharide and dexamethasone, and LPS+haMPCs-Exo is a group treated with lipopolysaccharide and extracellular vesicles of adipose mesenchymal progenitor cells; ^{∗∗∗} p < 0.001.
Figure 9 shows the inhibition of the release of pro-inflammatory factors in LPS-induced macrophages by extracellular vesicles of human-derived adipose mesenchymal progenitor cells. In the figure, (A) is a schematic flowchart of LPS induction of macrophages and treatment with extracellular vesicles of human-derived adipose mesenchymal progenitor cells, and (B) is the pro-inflammatory factor IL-6 released by macrophages and the level of protein TNF-α detected by ELISA. Control is the control group, LPS is a macrophage group treated with lipopolysaccharide alone, LPS+Dex is a group treated with lipopolysaccharide and dexamethasone, and LPS+haMPCs-Exo is a group treated with lipopolysaccharide and extracellular vesicle of adipose mesenchymal progenitor cells; ^{∗∗} p < 0.01, and ^{∗∗∗} p < 0.001.
Figure 10 is a schematic diagram of the cross-linking of extracellular vesicles, soluble cytokines and PEG to form hydrophilic particles.
Figure 11 shows a schematic process route of the present invention (from step S4 to step S8).

### Specific embodiments

Through extensive and in-depth research and repeated selection and studies, the inventors unexpectedly discovered for the first time a type of cell-free biopharmaceutical formulation that can effectively treat ARDS. The biopharmaceutical formulation contains extracellular vesicles derived from human cells that have specific particle sizes and are modified with a specific PEG; therefore, it can not only be stably stored for a long time, but also has excellent dispersibility in medical solvents (such as saline, etc.), making it especially suitable for direct administration to the lungs (especially the lower respiratory tract) and other parts of patients by means of atomized inhalation to quickly and efficiently treat or relieve the symptoms of pneumonia, ARDS and other diseases, and significantly reduce the risk of infection symptoms and mortality caused by coronaviruses, etc. Hence the present invention.

### Coronaviruses

Coronaviruses (CoVs) belong to the family Coronaviridae of the order Nidovirales. They are enveloped positive-strand RNA viruses. The subfamilies include the four genera, α, β, δ and γ.

Among the currently known human-infecting coronaviruses, HCoV-229E and HCoV-NL63 belong to genus α, while HCoV-OC43, SARS-CoV, HCoV-HKU 1, MERS-CoV and SARS-CoV-2 all belong to genus β.

The highly pathogenic coronaviruses "severe acute respiratory syndrome" or SARS-CoV and "Middle East respiratory syndrome" or MERS-CoV, which broke out in 2003 and 2012 respectively, are both genus β coronaviruses. The new coronavirus (SARS-CoV-2) that broke out at the end of 2019 is approximately 80% similar to SARS-CoV and 40% similar to MERS-CoV and is also a genus β coronavirus.

### Extracellular vesicles

### 1. Biological characteristics of the extracellular vesicles of mesenchymal stem cells

There are three major types of extracellular vesicles (EVs), i.e., exosomes, microvesicles, and apoptotic bodies. All the three major types of EVs are lipid bilayer delimited, and their diameters range between 30 and 2,000 nm.

Exosomes are a subtype of EVs that are derived from endosomes and have diameters of 50-100 nm. They are a main component of paracrine factors of a variety of cells including mesenchymal stem cells (MSCs).

MSC exosomes are a type of EVs derived from MSCs, with diameters ranging between 50 and 100 nm, and they are EVs with a complete lipid bilayer structure.

Exosomes are a type of vector that carries a variety of cargos, and their function comes into play mainly by continuously transferring miRNAs and proteins. In MSC-derived exosomes, more than 150 miRNAs and 850 distinctive proteins have been identified to change various activities of the target cells through different pathways. MSC exosomes are involved in physiological and pathological processes such as body development, epigenetic regulation, immune regulation (miR-155 and miR-146), tumorigenesis and tumor progression (miR-23b, miR-451, miR-223, miR-24, miR-125b, miR-31, miR-214 and miR-122), etc. According to ExoCarta data, over 900 types of proteins have been collected from MSC exosomes. Some studies have shown that MSC exosomes contain some cytokines and growth factors, such as TGF β1, interleukin-6 (IL-6), IL-10, hepatocyte growth factor (HGF), etc., which have been confirmed to be helpful in immune regulation. Vascular endothelial growth factor (VEGF), extracellular matrix metalloproteinase inducer (EMMPRIN) and MMP-9 have been reported in MSC exosomes. These three proteins play an important role in stimulating angiogenesis and may be the basis of exosomal tissue repair.

### 2. Biodistribution of the extracellular vesicles of mesenchymal stem cells in animals

In different animal models, several in vivo tracing strategies have been employed to determine the biodistribution of EVs or exosomes following their systemic administration. Near-infrared (NIR) dyes are ideal for in vivo applications thanks to their high signal-to-noise ratios. EVs labeled with superparamagnetic iron oxide nanoparticles have high-resolution and sensitive magnetic resonance analysis capabilities, providing a basis for accurate detection in deep organs.

In a rat model with intracerebral hemorrhage, DiI-labeled MSC exosomes were shown to reach the brain, liver, lung, and spleen after intravenous injection. In a mouse model with acute kidney injury (AKI), DID-labeled EVs accumulated specifically in the kidneys of the AKI mouse compared to healthy controls, indicating that exosomes appear to be able to home to sites of injury.

Nasal administration has led to better accumulation of brain exosomes at injury sites compared to intravenous administration. The biodistribution of EVs via systemic administration is a dynamic process: within approximately 30 minutes after administration, EVs rapidly distribute in the liver, spleen, and lungs, then enter the elimination phase by liver and kidney processing, and are cleared from 1 to 6 hours after administration.

### 3. Immunomodulatory and anti-inflammatory effects of the extracellular vesicles of mesenchymal stem cells

Studies have shown that exosomes play a key role in regulating tumor-specific T cell activation by carrying and presenting functional MHC peptide complexes.

Exosomes released from bone marrow-derived MSCs can inhibit the activation and infiltration of CD4-positive T cells, reduce the production of pro-inflammatory cytokines, promote the generation of IL-10-expressing Tregs, and inhibit Th17 cells, thereby effectively improving chronic graft-versus-host disease (cGVHD) in mice.

EVs derived from human pluripotent stromal cells have exhibited autoimmune-suppressing in models of type 1 diabetes (T1D) and experimental autoimmune uveoretinitis (EAU). EVs can inhibit the activation of antigen-presenting cells (APCs), inhibit Th1 and inhibit the development of Th17 cells, and increase the expression of the immunosuppressive factor IL-10.

Exosomes of human bone marrow MSCs can promote the proliferation of regulatory T cell subsets and enhance the immunosuppressive ability of asthma patients by up-regulating the expression of cytokine IL-10 and TGF-β1 in peripheral blood mononuclear cells (PBMCs).

The miR-181c in exosomes derived from human umbilical cord MCSs exerted an anti-inflammatory effect in a rat model with inflammation from burns by down-regulating the TLR4 signaling pathway.

In a porcine model with renal artery stenosis, a single intrarenal injection of porcine autologous adipose MSCs-EVs could reduce the levels of various pro-inflammatory cytokines (TNF-α, IL-6, and IL-1-β) in the renal veins while inhibiting the increase of the inflammatory factor IL-10, accompanied by migration of macrophages from pro-inflammatory cells to repairing macrophages, again demonstrating the immunomodulatory potential of EVs.

Many studies have reported the application of MSCs in animal models with liver fibrosis/cirrhosis and acute liver injury, which could ultimately improve the disease progression in patients. Exosomes of adipose MSCs could significantly reduce the elevation of serum alanine aminotransferase and aspartate aminotransferase levels in a concanavalin A (Con A)-induced hepatitis model of C57BL/6 mice, alleviating liver inflammation, and reducing the severity of hepatitis in the hepatitis model. The levels of pro-inflammatory cytokines (TNF-a, IFN-γ, IL-6, IL-18, and IL-1 β) in the serum of the hepatitis model of mice were reduced, and the activation of inflammasomes in the liver of the hepatitis model of mice was inhibited.

Sepsis is a systemic inflammatory response of the body against microbial infections. Despite the application of advanced antibiotics, sepsis mortality remains high in intensive care units, and therefore researchers have targeted MSCs in the treatment of this systemic inflammatory disease. In recent years, the efficacy of MSC-EVs in animal models of cecal ligation-induced sepsis has been extensively studied. Intravenous treatment with adipose MSCs-EVs relieved systemic inflammation, organ damages, and subsequent lethality in a rat model of sepsis. In another study of the treatment of sepsis, EVs derived from MSCs pretreated with IL-1β were significantly more effective in the treatment of sepsis than EVs derived from MSCs without the pretreatment.

Studies have also showed that EVs derived from induced MSCs could efficiently polarize macrophages to differentiate into type M2, an anti-inflammatory phenotype of macrophages. As an anti-inflammatory miRNA, miR-146a was significantly increased in both IL-1β-pretreated MSCs and EVs. Transfer of miR-146a packaged in EVs into macrophages could polarize them to type M2 [Casado JG, et al. Frontiers in Veterinary Science. 2017; 4:39].

### 4. Genetically modified MSCs can increase the efficacy of extracellular vesicles thereof

To improve the migration of human bone marrow MSCs to the lung and enhance the therapeutic potential of MSCs, an AT2R DNA-containing construct which had high and stable long-term expression in bone marrow MSCs was obtained by lentivirus-mediated gene transfection. The migration of AT2R-MSCs was detected in vitro. Subsequently, the potential therapeutic efficacy of AT2R-MSCs was investigated in an in vivo model of lipopolysaccharide (LPS)-induced acute lung injury, and the migration of MSCs to injured lung tissue was assessed. At the same time, AT2R knockout MSCs (MSC-ShAT2R) were used to determine the role of AT2R in the migration ability of MSCs in vivo and the therapeutic effect on ARDS.

MSCs exosomes alleviated lung ischemia/reperfusion injury in mice by transporting anti-apoptotic miR-21-5p. The miR-21-5p in exosomes reduced oxidative stress-induced apoptosis by targeting PTEN and PDCD4 in lung tissue.

Recent studies have found that exosomes derived from MSCs overexpressing miR-30b-3p could inhibit serum amyloid A3 (SAA3).

In models of cecal ligation and perforation-induced sepsis, exosomal miR-146a enhanced the therapeutic effect of IL-1β-pretreated MSCs, and it was further found that IL-1β stimulation of MSCs could up-regulate the expression of miR-146a in MSCs, which could be packaged in exosomes. This exosomal miR-146a was then transferred to macrophages, leading to M2 polarization and ultimately to increased survival in septic mice. Therefore, modifying the exosomes of MSCs by overexpression of specific miRNAs is a promising new direction for developing ARDS therapies.

### Acute respiratory distress syndrome

ARDS is an acute systemic inflammatory response to direct or indirect lung injury due to factors such as smoking, drowning, aspiration, sepsis, trauma, ischemia, exposure to toxins, etc. Severe inflammatory responses will cause changes in vascular permeability, leading to acute pulmonary edema.

The pathological process of ARDS mainly has three stages: the exudative stage, proliferative stage and fibroproliferative stage.

During the exudative stage, the inflammatory cascade caused by lung injury and the dysfunction of the alveolar-capillary barrier result in increased permeability of alveolar epithelial and pulmonary capillary endothelial cells, which is characteristic of the exudative stage. Pathological manifestations of lung tissue include diffuse alveolar damage with exudation, microvascular damage with secondary pulmonary edema, necrosis of alveolar type 1 (AT1) epithelial cells, aggregation of inflammatory cells, and release of active mediators. Alveolar inflammation is mainly caused by polymorphonuclear neutrophils, monocytes, and macrophages. Other pro-inflammatory mechanisms are also involved, for example, the release of large amounts of pro-inflammatory cytokines from lung cells, inflammatory cells and fibroblasts.

During the proliferative stage, hyaline membranes form within the alveoli with infiltration of inflammatory cells, including T lymphocytes, neutrophils, and macrophages. Extracellular matrix deposits in the alveoli following a severe damage from inflammation and oxidative stress, with persistent chronic inflammation. Inflammation cascade plays a key role in apoptosis, proliferation, migration and other processes closely related to ARDS. Damages that are persistent and cannot be repaired in time are the main pathological manifestations of ARDS in the fibroproliferative stage.

During this fibroproliferative stage, fibroblast proliferation, AT2 cell proliferation and lung tissue repair will take place. The repair mechanism of damaged alveolar epithelium is not fully understood, but it involves the proliferation of AT2 cells, which migrate along the basement membrane to form a new epithelial barrier and have complex interactions with the extracellular matrix and other cells including alveolar macrophages, resulting in significant changes in the lung structure and functions in some cases. Computed tomography (CT) of the lung can detect dense fibrosis and honeycomb structures during the fibroproliferative stage of ARDS.

Severe diseases associated with ARDS pose a high risk of ventilator-acquired pneumonia, acute myocardial infarction, and acute pulmonary embolism.

### Extracellular vesicles

The present invention provides an extracellular vesicle derived from mesenchymal stem cells (including adipose mesenchymal stem (progenitor) cells, umbilical cord mesenchymal stem cells and placental amniotic membrane mesenchymal stem cells).

In the present invention, the extracellular vesicles prepared by the specific optimized process of the present invention have specific particle size distributions, and are cross-linked with PKG to form hydrophilic particles, which are not only extremely suitable for administration and action in the form of an atomized inhalation preparation in the lung (especially the lower respiratory tract), but also features high stability in aqueous solutions for a long time and excellent dispersibility (both superior to the original cells).

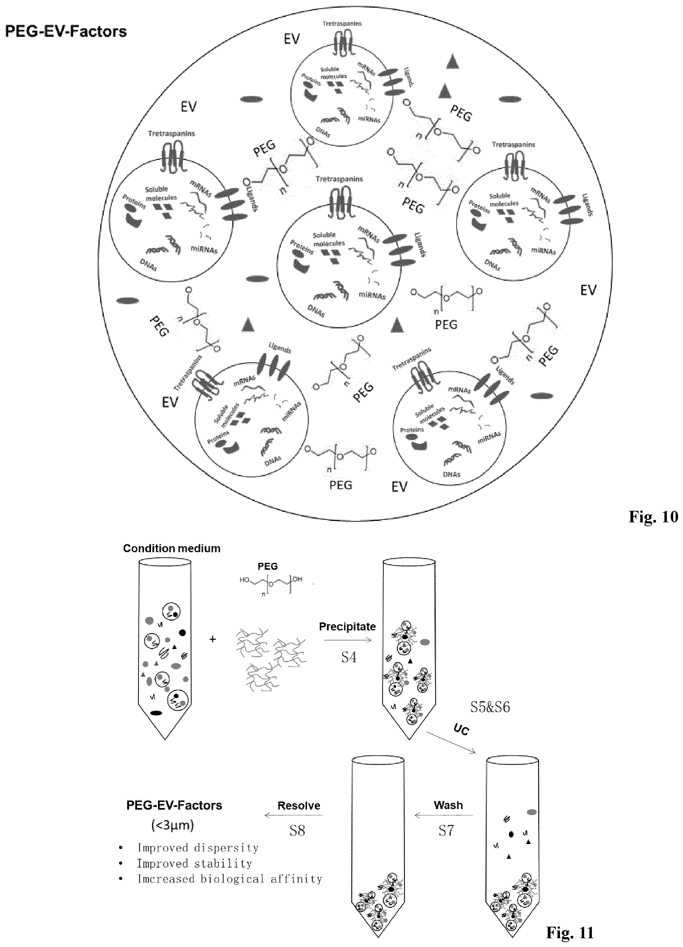

For ease of understanding, the applicant provides a schematic diagram in Figure 10. As shown in Figure 10, extracellular vesicles, soluble cytokines and PEG cross-link to form hydrophilic particles.

In another preferred example, the particle sizes of the extracellular vesicles obtained by the separation method and process described in the present invention are preferably 50-500 nm. Extracellular vesicles, soluble cytokines and PEG cross-link to form hydrophilic particles with particle sizes of less than 3 microns (for example, approximately 1-3 microns or 0.5-3 microns), which have better stability and dispersibility in aqueous solutions than the cells themselves. The hydrophilic particles formed by cross-linking of extracellular vesicles, soluble cytokines and PEG are mixed with solutions such as saline, artificial tears, and hyaluronic acid to respectively form atomized inhalation liquid, eye drops, nasal drops and an external use formulation, which is beneficial for local mucosal cells to absorb and utilize these extracellular vesicles and soluble active cytokines. Their biodistributions in vivo are consistent with those of pure extracellular vesicles.

In the present invention, it is advisable to use mesenchymal stem cells to prepare the cells of extracellular vesicles. Representative examples of mesenchymal stem cells include (but are not limited to): adipose mesenchymal stem cells, umbilical cord mesenchymal stem cells, placental amniotic mesenchymal stem cells, or a combination thereof.

### Pharmaceutical composition and atomized inhalation formulation

The present invention also provides a pharmaceutical composition, which contains (a) an active ingredient derived from human somatic cells, which is extracellular vesicles produced by human somatic cells; and (b) a pharmaceutically acceptable carrier.

In another preferred example, the pharmaceutical composition is an injection or a cell-free pharmaceutical composition.

In the present invention, preferably, the active ingredient further comprises soluble active cytokines, and these soluble cytokines are mainly present in extracellular vesicles. In addition, some soluble cytokines may exist outside extracellular vesicles. Some soluble cytokines may also be modified by PEG.

In the present invention, a particularly preferred pharmaceutical formulation is an atomized inhalation formulation.

### Preparation method

The present invention also provides a method for preparing the extracellular vesicles.

A typical method is to prepare the extracellular vesicles by use of mesenchymal stem cells (including adipose mesenchymal stem (progenitor) cells, umbilical cord mesenchymal stem cells, and placental amniotic membrane mesenchymal stem cells) as a raw material.

Taking adipose mesenchymal stem (progenitor) cells as an example, a typical preparation method comprises the following steps:
(S1) Culturing cells (for example, adipose mesenchymal stem (progenitor) cells) to a predetermined confluency (for example, 75-90%);
(S2) Under conditions suitable for EV production, continuing the culture of the cells for a period of time T1; T1 is usually 24-72 hours, preferably 30-60 hours;
(S3) Removing the cells from the culture system to separate and obtain a culture medium containing extracellular vesicles, i.e., "conditioned medium";
(S4) Mixing the conditioned medium with polyethylene glycol (PEG) to form a first mixture, and placing it for a period of time T2, thereby forming PEG-modified extracellular vesicles; T2 is usually 6-60 hours, preferably 12-48 hours;
(S5) Centrifuging the first mixture from the previous step to precipitate the PEG-modified extracellular vesicles, and removing the supernatant to obtain a PEG-modified extracellular vesicle precipitate;
(S6) Resuspending the PEG-modified extracellular vesicle precipitate obtained from the previous step to obtain a first resuspension mixture;
(S7) Centrifuging the first resuspension mixture to precipitate the PEG-modified extracellular vesicles, and removing the supernatant to obtain a PEG-modified extracellular vesicle precipitate;
(S8) Resuspending the PEG-modified extracellular vesicle precipitate obtained from the previous step to obtain a medically acceptable extracellular vesicle formulation (i.e., PEG-EVs-factors formulation).

A schematic process route from steps S4 to S8 is shown in Figure 11.

### Scale of the production process for extracellular vesicles under GMP conditions

One HyperFlask cell factory can produce extracellular vesicles in 500 ml of the conditioned medium, and it is estimated that a total of 5 × 10¹⁰-10 × 10¹⁰ extracellular vesicles can be obtained by separation.

With the current laboratory capacity, one production operation unit can separate a total of 800-1200 ml of conditioned medium from two cell factories to obtain an estimated total of 2-5 × 10¹¹ extracellular vesicles, and one batch produced from one production operation unit can meet the need of the local usage for 100-250 patients if a total of 2-5 × 10⁹ extracellular vesicles are used locally for each patient.

### Main advantages of the present invention include:

(a) The present invention is suitable for scale production, especially for clinical industrial scale production. One HyperFlask cell factory can produce extracellular vesicles in 500-600 ml of the conditioned medium, and it is estimated that a total of 2-5 × 10¹¹ extracellular vesicles can be obtained by separation. With the GMP laboratory capacity, one production operation unit can separate 800-1,200 ml of the conditioned medium from two cell factories to obtain an estimated total of 2-5 × 10¹¹ separated and purified extracellular vesicles. One batch produced from one production operation unit can meet the need of 100-250 patients if a total of 2-5 × 10⁹ extracellular vesicles are used locally for each patient.
(b) Good stability: There is no need of antifreezing liquid such as DMSO, which is harmful to human body. It can be stored for 36 months or longer at low temperature (-20°C) and ultra-low temperature (-80°C to -196°C), and its membrane structure and the contents are stable.
(c) High dispersibility: Good dispersibility in pharmaceutically acceptable carriers, especially isotonic aqueous sodium chloride solution. After freezing and thawing, there is no crystallization or precipitation when it is placed at 4°C for 24 hours or more, which is significantly better than cell-based formulations.
(c) The pharmaceutical composition of the present invention has hydrophilic particles smaller than 3 microns and is uniformly dispersed in an aqueous solution, which, when administered by atomization and inhalation, can enter the respiratory tract, especially the lower respiratory tract, with unusually high efficiency.

The present invention is further described by referring to some embodiments. It should be understood that the embodiments are only used to illustrate the present invention but not to limit the scope of the present invention. In the following embodiments where no conditions are specified for the experimental method, the conventional conditions are generally used, for example, the conditions described in Sambrook et al., Molecular Cloning: A Laboratory Manual (New York: Cold Spring Harbor Laboratory Press, 1989). Unless otherwise specified, percentages and parts are weight percentages and parts by weight.

### Materials

Adipose mesenchymal stem (progenitor) cells: taking fat by abdominal liposuction or abdominal dermolipectomy from a healthy male or female volunteer who has passed ethical review, has been registered, has signed the informed consent, and has been strictly examined by a laboratory to meet the criteria inclusion into the adipose stem (progenitor) cell bank, placing the fat in a cell storage solution, transporting it at a low temperature to a qualified GMP laboratory, and obtaining adipose mesenchymal progenitor cells, i.e., working bank cells (intermediate product), by separation, purification and proliferation.

### General methods

### 1. Medium pretreatment

The α MEM medium containing 5% EliteGro was centrifuged under 120,000 g at 4°C for 6 h.

### 2. Obtaining extracellular vesicles by PEG precipitation

12% PEG 6000 was prepared with PBS, filtered with a 0.22 µm filter, added to the treated conditioned medium at a volume ratio of 1:1, incubated at 4°C overnight, and centrifuged under 3,000 g at 4°C for 1 h, and the supernatant was removed. Pre-cooled PBS was added to resuspend the precipitate, ultracentrifugation was performed under 12,0000 g at 4°C for 70 minutes, the supernatant was sucked out, and an appropriate amount of isotonic sodium chloride solution was added to resuspend the precipitate to obtain the extracellular vesicles.

### 3. Measurement of particle diameter distribution and particle concentration

An appropriate amount of the sample was taken, diluted by 300 times, and Zeta View was used to measure the particle diameter distribution and particle concentration of the sample.

### 4. Measurement of protein concentration

An appropriate amount of the sample was taken, and the protein concentration was measured by BCA. 5 × SDS loading buffer was added, and the sample was heated at 100°C for 5 minutes A sample with the same amount of protein was loaded onto a SDS polyacrylamide gel, and electrophoresis was performed at 100 V until the dye migrated to the gel bottom. At 250 mA, the sample was transferred to a PVDF membrane after 90 minutes TBST buffer was mixed with 5% skim milk as the blocking buffer and the primary and secondary antibody dilution buffers. The PVDF was blocked. The samples were respectively incubated with the primary antibody dilution buffers, i.e., positive protein CD63, CD81, TSG101, and negative protein CANX, and incubated with the corresponding secondary antibody dilution buffers. Then the developing solution was added, and the protein bands were observed.

### Example 1

### Preparation of the medium containing extracellular vesicles (conditioned medium)

2 × 10⁷ adipose mesenchymal stem cells (passage P4) were taken, seeded in a basic medium α MEM containing 5% EliteGro, and cultured in a HyperFlask culture flask under the culture conditions of 37°C and 5% CO₂.

When the cell confluency reaches approximately 80%, the medium was changed to the pretreated medium for culturing under the culture conditions of 37°C and 5% CO₂ for 48 h.

The supernatant was collected and centrifuged under 3,000 g for 15 minutes, and the supernatant was taken and centrifuged under 10,000 g for 30 minutes at 4°C. The supernatant was taken as the medium containing extracellular vesicles ("conditioned medium" hereinafter).

### Example 2

### Preparation of extracellular vesicles

In this example, the extracellular vesicles were prepared by PEG precipitation and ultracentrifugation, respectively.

### Ultracentrifugation:

The conditioned medium (from example 1) was taken and ultracentrifuged under 120,000 g at 4°C for 70 minutes. After resuspending with pre-cooled PBS, it was ultracentrifuged again under 120,000 g for 70 minutes. The precipitate as resuspended with an appropriate amount of isotonic sodium chloride solution to obtain the extracellular vesicles.

### PEG precipitation:

PBS was used to prepare PEG 6000 of the concentration of 8%, 12%, 16% or 20%, which was filtered with a 0.22 µm filter, added to the treated conditioned medium (from example 1) at a volume ratio of 1:1, and incubated at 4°C overnight.

Centrifugation was performed under 3,000 g at 4°C for 1 hour, and the supernatant was removed. Pre-cooled PBS was added to resuspend the precipitate, ultracentrifugation was performed under 120,000 g at 4°C for 70 minutes, the supernatant was removed, and an appropriate amount of isotonic sodium chloride solution was added to resuspend the precipitate to obtain the extracellular vesicles.

### Performance measurement:

The separated extracellular membrane vesicles containing 8% PEG 6000, 12% PEG 6000, 16% PEG 6000, and 20% PEG 6000 were loaded to the same total protein amount, and CD63 determination was performed.

The results are shown in Figure 1A. The CD63 expression levels of the extracellular membrane vesicles containing 12% PEG 6000 and 16% PEG 6000 were the highest. Therefore, 12% PEG 6000 was used in subsequent experiments.

In addition, the extracellular vesicles obtained by separating 200 ml of the medium with 12% PEG 6000 and the extracellular vesicles obtained by separating 350 ml of the medium by ultracentrifugation were loaded to the same volume and the same amount of protein for CANX, TSG101 and CD81 determination and for NTA detection.

The results are shown in Figures 1B to 1D. The results showed that the amounts of the target proteins of the extracellular vesicles separated by PEG 6000 were much higher than those of the extracellular vesicles separated by ultracentrifugation, when detected by Western blot with the same volume or the same protein amount.

The NTA results showed that the concentration of extracellular vesicles separated by PEG 6000 was 1.35 × 10⁸ extracellular vesicles/ml medium, and the concentration of extracellular vesicles separated by ultracentrifugation was 6.8 × 10⁷ extracellular vesicles/ml medium.

The particle size of the extracellular vesicles separated by PEG 6000 was 126.6 ± 2.09 nm, and that of the extracellular vesicles separated by ultracentrifugation was 137.8 ± 3.8 nm.

These experimental results validate that the yield of extracellular vesicles separated by 12% PEG 6000 was much higher than that by ultracentrifugation, and that PEG did not affect the particle size of extracellular vesicles.

### Example 3

### Intake of extracellular vesicles by fibroblasts

PKH-67 was used to label the extracellular vesicles separated by PEG 6000.

Figure 2 shows that the extracellular vesicles separated by PEG 6000 did not affect the intake of extracellular vesicles by fibroblasts.

### Example 4

### Determination of stability and performance of extracellular vesicles

The extracellular vesicles prepared by separation were mixed with saline to prepare aqueous solutions, which were stored at 4°C, -20°C and -80°C respectively for 9 weeks. After 9 weeks, the extracellular vesicles of the 3 temperatures were tested by NTA and their performance was determined.

The results of the NTA detection are shown in Figure 3 and Table 1.

**Table 1 Particle number detected by NTA**

| Unit: (10¹⁰ extracellular vesicle particles/ml) | | | |
|---|---|---|---|
| Primary separation | Stored for 9 weeks | | |
| Primary separation | 4°C | -20°C | -80°C |
| 1.3 | 1.5 | 1.6 | 1.8 |
| 1.2 | 1.4 | 1.5 | 1.8 |
| 1.2 | 1.4 | 1.6 | 1.8 |

The results showed that the number of extracellular vesicles stored at the three different temperatures for 9 weeks did not significantly decrease in the number of particles compared with those on day 0 after primary separation (Figure 3).

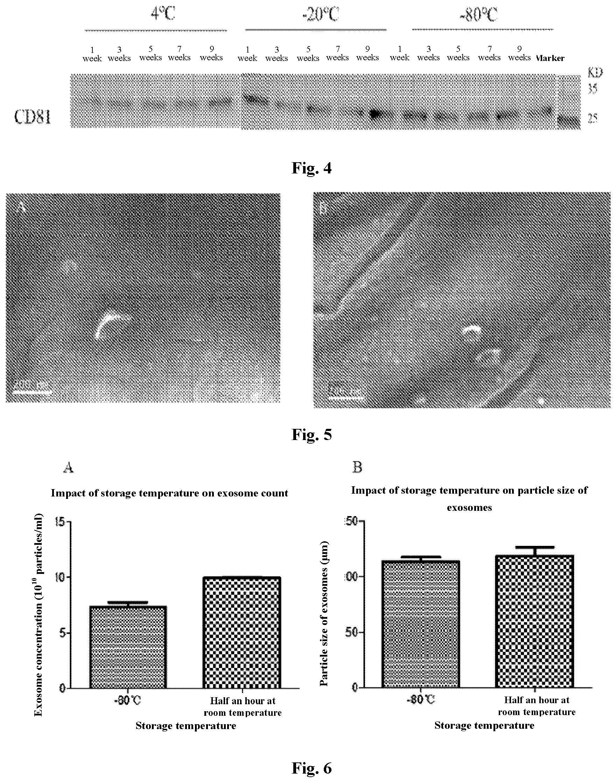

The results of Western protein electrophoresis showed that the specific markers CD81 and TSG101 of the extracellular vesicles stored at -20°C and -80°C for 9 weeks were stably expressed. At 4°C, the specific marker TSG101 of the extracellular vesicles stored for 3 weeks could be stably expressed, while the expression of this marker began to decrease from the 5th week (Figure 4).

The extracellular vesicles stored at -80°C for 1 week were "saucer-like" under a transmission electron microscope (Figure 5A). After half an hour of thawing at room temperature, the extracellular vesicles were still "saucer-like" under the transmission electron microscope (Figure 5B), and there was no significant change.

The extracellular vesicles stored at -80°C for 1 week and the extracellular vesicles thawed at room temperature for half an hour were detected by NTA, and it was found that there was no significant difference in the particle concentration (Figure 6A) and size (Figure 6B).

### Example 5

### Detection of multiple cytokines in a lyophilized formulation containing extracellular vesicles and cytokines

In this example, cytokines were detected for the extracellular vesicles prepared by the method of the present invention. The tested samples included:
Sample 1: lyophilized powder of the basal medium (negative control), stored at 4°C for 4 weeks;
Sample 2: lyophilized powder of the stem cell medium (positive control), stored at 4°C for 4 weeks;
Sample 3: lyophilized powder of the extracellular vesicles, stored at room temperature for 4 weeks;
Sample 4: lyophilized powder of the extracellular vesicles, stored at 4°C for 4 weeks; and
Sample 5: lyophilized powder of the extracellular vesicles, stored at -20°C for 4 weeks.

The detection results of a plurality of cytokines are shown in Table 2.

**Table 2 Cytokine detection results**

| | **PDGF-BB (pg/ml)** | **MMP-2 (pg/ml)** | **EGF (pg/ml)** | **VEGF (pg/ml)** | **FGF (pg/ml)** | **HGF (pg/ml)** |
|---|---|---|---|---|---|---|
| Sample 1 | < 1.96 | 339.41 | < 1.50 | 5.24 | 10.09 | 3.63 |
| Sample 2 | 9501.00 | 26600.00 | 7.80 | 1243.00 | 28.59 | 49.74 |
| Sample 3 | 34.92 | 23195.00 | 7.80 | 1243.00 | 28.59 | 49.74 |
| Sample 4 | 91.91 | 22960.00 | 10.95 | 2161.00 | 53.15 | 87.98 |
| Sample 5 | 94.00 | 21276.00 | 10.62 | 2164.00 | 53.96 | 92.82 |

The results showed that the extracellular vesicles prepared by the separation and purification method described in the present invention were rich in cytokines. The cytokines stayed stable when the samples were stored at -20°C and -4°C for 4 weeks.

### Example 6

### Inhibition of LPS-induced macrophage activation by extracellular vesicles of human-derived adipose mesenchymal progenitor cells

To verify that extracellular vesicles of human-derived adipose mesenchymal progenitor cells have anti-inflammatory effects on lipopolysaccharide (LPS)-induced inflammation in mouse Raw264.7 macrophages, in this example, LPS-induce inflammation in macrophages was used as the model to study the effect of extracellular vesicles of human-derived adipose mesenchymal progenitor cells on its activation level in vitro. The specific method is as follows:
Firstly, four experiment groups were set up, specifically, a control group without any drug treatment, a group treated with 0.1 µg/ml LPS alone, a group treated with 0.1 µg/ml LPS and 5 µg/ml dexamethasone (Dex), and a group treated with 0.1 µg/ml LPS and extracellular vesicles of human-derived adipose mesenchymal progenitor cells of an appropriate concentration (haMPCs-Exo).

1 ml of Raw264.7 macrophage suspension was added to each well of a 12-well culture plate (1 × 10⁴ cells/well), which was placed in a CO₂ constant temperature incubator (with 5% CO₂, at 37°C) for 1 h. Then, the samples were treated to form the above groups and were cultured in a CO₂ constant temperature incubator (with 5% CO₂, at 37°C) for 4 h. Lastly, the cultured plate was taken out, the morphological changes of activated mouse Raw264.7 macrophages were observed under an inverted microscope, and a field of view under a 20x objective lens was randomly selected for photographing, recording and statistical analysis.

The results are shown in Figure 7. Compared with the control group, the activated macrophages (pro-inflammatory macrophages) were branched after induction with LPS alone for 4 hours (Figure 7A), and statistical analysis found that the activation quantity was also significantly increased (Figure 7B), indicating that an in vitro LPS-induced macrophage inflammation model was successfully established.

Compared with the group treated with LPS alone, the number of branched macrophages was significantly reduced in the group treated with LPS and dexamethasone for 4 hours (Figure 7A), and the number of activated macrophages was significantly lower in statistical analysis (Figure 7B).

Similarly, after 4 hours of treatment with LPS and extracellular vesicles of human-derived adipose mesenchymal progenitor cells, the number of branched macrophages was also significantly reduced (Figure 7A), and the number of activated macrophages was also significantly lower in statistical analysis (Figure 7B).

Therefore, these results suggest that extracellular vesicles of human-derived adipose mesenchymal progenitor cells could inhibit LPS-induced macrophage activation, which in turn impaired the formation of pro-inflammatory macrophages.

### Example 7

### Inhibition of the gene expression of pro-inflammatory factors in LPS-induced macrophages by extracellular vesicles of human-derived adipose mesenchymal progenitor cells

In this example, to verify that extracellular vesicles of human-derived adipose mesenchymal progenitor cells have an inhibitory effect on the inflammatory response of LPS-induced mouse Raw264.7 macrophages, real-time quantitative PCR was used to detect the gene expression of the main inflammatory factors. The specific operation method is essentially the same as that described in example 1, except that the drug treatment was performed for 24 hours. The total RNA extraction was performed according to the instructions of the Beyotime RNA Isolation Kit, while the real-time fluorescence quantitative PCR detection was performed with the TaqMan probe for simultaneous detection of the target genes and the internal reference gene, and the relative quantitative analysis was carried out by the 2^{-ΔΔCT} method.

The results are shown in Figure 8. Compared with the control group, the gene expression levels of pro-inflammatory cytokines in mouse Raw264.7 macrophages were significantly increased after treatment with LPS alone for 24 hours. However, after treatment with LPS and dexamethasone for 24 hours, the expression levels of TNF-α, IL-1β and IL-6 were greatly decreased (Figures 8A, B and C).

Similarly, after treatment with LPS and extracellular vesicles of human-derived adipose mesenchymal progenitor cells for 24 hours, the expression levels of TNF-α, IL-1β and IL-6 were all significantly reduced, essentially reaching the levels of the group treated with dexamethasone (Figure 8A, B and C).

Therefore, these results suggest that the extracellular vesicles of human-derived adipose mesenchymal progenitor cells could significantly inhibit the gene expression levels of pro-inflammatory cytokines in LPS-induced macrophages, thereby inhibiting their inflammatory responses.

### Example 8

### Inhibition of the release of pro-inflammatory factors in LPS-induced macrophages by extracellular vesicles of human-derived adipose mesenchymal progenitor cells

In this example, to further verify that extracellular vesicles of human-derived adipose mesenchymal progenitor cells can inhibit the inflammatory response of LPS-induced mouse Raw264.7 macrophages, the ELISA method was used to detect the levels of the inflammatory factors they released. The specific operation method is the same as that described in example 2, except that the drug treatment was performed for 24 hours. Refer to Figure 9A for the experimental scheme. The operation of ELISA to detect inflammatory factors in macrophages was performed according to the instructions of Beyotime TNF-α and IL-6 kits, and then a statistical analysis was made.

The results show that, compared with the control group, pro-inflammatory cytokines TNF-α and IL-6 released by mouse Raw264.7 macrophages were both significantly increased after treatment with LPS alone for 24 hours (Figure 9B). However, after treatment with LPS and dexamethasone for 24 hours, the levels of TNF-α and IL-6 were greatly decreased (Figures 9B).

Similarly, after treatment with LPS and extracellular vesicles of human-derived adipose mesenchymal progenitor cells for 24 hours, the levels of TNF-α and IL-6 were both greatly reduced, reaching the levels of the group treated with dexamethasone (Figure 9B). Among them, extracellular vesicles had a better inhibitory effect on IL-6, suggesting that they had better application prospects in intervention therapies that inhibit a cytokine storm in the novel coronavirus pneumonia.

Therefore, the above data suggests that extracellular vesicles of human-derived adipose mesenchymal progenitor cells could significantly inhibit the release of pro-inflammatory cytokines in LPS-induced macrophages, thereby inhibiting their pro-inflammatory responses.

### Example 9

### Scheme for studying biodistribution of fluorescently labeled EVs in nude mice by atomized inhalation

In this example, the distribution and duration of exosomes of human-derived adipose mesenchymal progenitor cells in the lung tissue of mice after atomized inhalation were studied. Fluorescence-labeled exosome tracing method was used in the study.

PKH67-labeled exosomes of human-derived adipose mesenchymal progenitor cells (1 × 10⁶ extracellular vesicles/g) were atomized for 30 minutes and inhaled into the lungs of nude mice (N = 3), the distribution of PKH67-labeled exosomes in the lung tissue of nude mice was observed by in vivo fluorescence imaging at 2 h, 4 h, 6 h, 8 h, 12 h and 24 h after inhalation, and the time of their retention in the lungs of nude mice was analyzed.

### Example 10

### Scheme for studying the efficacy of atomized inhalation of EVs in the treatment of infectious lung injury in an animal model

In this example, the efficacy of exosomes of human-derived adipose mesenchymal progenitor cells for the treatment of multidrug-resistant aeruginosa (PA)-induced pneumonia in a mice model after inhalation was studied. A blank control group of PBS-induced mice, a group of PA-induced mouse pneumonia, a group of PA-induced mouse pneumonia treated by exosomes of human-derived adipose mesenchymal progenitor cells through atomized inhalation, and a group of mouse pneumonia treated by mouse fibroblast exosomes were set up respectively. Each group consisted of six 8-10-week-old C57BL/6 wild-type male mice, and the amount of exosomes in each mouse in the treatment group was calculated to be 1×10⁶ extracellular vesicles/g. After 4 hours of multidrug-resistant aeruginosa induction, exosome inhalation was performed once a day for 5 days. Then, blood samples were collected for peripheral blood leukocyte and neutrophil counts and bacterial load measurement. Bronchoalveolar lavage fluid was collected for neutrophil count, bacterial load measurement and determination of related pro-inflammatory factor levels. In addition, the bacterial load in the lung tissue was measured, and the morphological observation of the lung tissue was performed by HE staining.

### Example 11

### Protocol of clinical trial of atomized inhalation of EVs for the treatment of acute lung injury from novel coronavirus pneumonia

In this example, the safety and efficacy of exosomes of allogeneic adipose-derived mesenchymal stem cells for the treatment of severely and critically ill patients with novel coronavirus pneumonia (NCP) by atomized inhalation were preliminarily studied.

It was planned to include 30 severe and critical NCP subjects, who would be divided into three dosage groups, i.e., low, medium and high dosages. In addition to routine treatment in ICU, all subjects were given atomized inhalation of allogeneic adipose MSCs-Exo as a combined therapy of 2-8 × 10⁸ extracellular vesicles/3 ml, which was administered once a day for 5 consecutive days. The incidence of adverse reactions and clinical improvement time within 28 days, as well as the number of patients weaned off, the number of ICU nursing days, the duration of mechanical ventilation of survivors and the mortality, etc., were recorded and analyzed. In addition, the improvement of symptoms in patients after treatment was evaluated by daily SOFA score, detection of relevant biochemical indicators and lung imaging observation, and the time of the coronavirus turning negative in patients' respiratory specimens. The inclusion criteria for this study mainly included 1) male or female who was 18-75 years old and who or the family members of whom voluntarily took part in the study and signed the informed consent; 2) who was tested positive in a RT-PCR test or diagnosed with novel coronavirus pneumonia; and 3) who met the diagnostic criteria for severely and critically ill patients. The exclusion criteria mainly included 1) related virus carriers or patients who had severe allergy, and patients whose pneumonia was caused by other viruses; 2) patients with lung cancer or long-term use of immunosuppressive drugs; 3) patients receiving hemodialysis or peritoneal dialysis, or with abnormal liver function; 4) patients who were using ECMO or high-frequency oscillatory ventilation; 5) patients who were pregnant, breastfeeding or planning to conceive within six months; 6) patients who were assessed by the investigators to be unable to participate in the study or have failed to understand and implement the protocol.

### Discussion

For the treatment of acute respiratory distress syndrome, some alternative cell therapies include the embryonic stem cells (ESCs) therapy, in vitro induced pluripotent stem cells (iPSCs) therapy, mesenchymal/stromal stem cells (MSCs) therapy, pulmonary epithelial progenitor cells (EpPCs) therapy, endothelial progenitor cells (EnPCs) therapy, etc.

The MSCs therapy is the most studied stem cell therapy. MSCs are self-renewing and proliferating pluripotent stem cells that can suppress immune responses in vitro and have the potential to differentiate into alveolar type 2 cells (AT2 cells). MSCs-mediated inflammation suppression and MSC-related lung repair and regeneration are the main mechanisms of their potential for the treatment of pulmonary diseases such as ARDS, pneumonia, asthma, chronic obstructive pulmonary disease (COPD), interstitial pulmonary fibrosis (IPF), etc.

As mentioned previously, ARDS is a severe clinical syndrome resulting from disruption of the alveolar epithelial barrier, accompanied by interstitial edema and inflammatory cell infiltration leading to progressive acute respiratory failure. Although synthetic corticosteroids, surfactants, inhaled nitric oxide, antioxidants, protease inhibitors, and various other anti-inflammatory treatments such as simvastatin and ibuprofen have been used to treat ARDS, none of these drugs significantly reduces ARDS mortality, which has remained at 34%-44%.

Several recent preclinical studies have demonstrated that MSCs, along with their paracrine cytokines and extracellular microvesicles, may be considered as a novel and effective treatment for ARDS. ARDS is often a complication of severe sepsis, especially after Gram-negative bacteria infection. Treatment with MSCs prevented ARDS in an animal model of Escherichia coli-derived lipopolysaccharide (LPS)-induced sepsis [Curley GF, et al. Critical Care Med. 2017, 45(2): e202-e212] [Lee JW, et al. Stem Cell 2011, 29(6): 913-919].

Intravenous injection of MSCs could significantly improve alveolar damage and inflammatory response in a lipopolysaccharide-induced ARDS mouse model. MSCs reduced the infiltration of neutrophils in the lung and the production of the inflammatory factor TNF-α by immune cells infiltrating the lung in a paracrine and interleukin-10 (IL-10)-dependent manner [Gupta N, et al. Journal of Immunology 2007, 179, (3): 1855-1863] [Mei SHJ, et al. PLoS Medicine, 2007, 4(9): e269].

In addition, through the production of the keratinocyte growth factor (KGF), vascular endothelial growth factor (VEGF), and hepatocyte growth factor (HGF), MSCs promoted AT type 2 cell regeneration, prevented endothelial cell apoptosis, and promoted repair of the lung epithelial barrier in ARDS injury [Lee JW, et al. Proceedings of the National Academy of Sciences of the United States of America 2009, 106(38): 16357-16362] [Hu S, et al. Stem Cell Research & Therapy 2016 , 7(1): 66]. Reduced inflammatory damage, reduced edema, improved oxygenation, and prolonged survival were observed in an animal model treated with MSCs. MSCs protect themselves from sepsis-associated ARDS in a PGE-2-dependent manner by increasing the production of the anti-inflammatory factor IL-10 in macrophages.

Early clinical trial of intravenous infusion of bone marrow MSCs for ARDS has been completed. [J. G. Wilson JG, et al. Lancet Respir. Med. 2015, 3(1): 24-32] [Zheng G, et al. Respir. Res. 2014, 15(1): 39]. Wilson et al. reported that in 9 patients with moderate to severe ARDS, good tolerance of a single intravenous infusion of allogeneic bone marrow MSCs (1 million, 5 million, and 10 million cells/kg) was observed in a clinical study (NCT01775774). No adverse reactions and dose-limiting toxicities were observed in the 9 patients treated with allogeneic bone marrow MSCs. Following the conclusion of this phase I clinical study, a randomized multi-center phase II clinical study was conducted in the United States from 2014 to 2018 (XCT02097641). The study enrolled 60 adult ARDS patients who were treated with a single dose of allogeneic bone marrow MSCs (10 million cells/kg) or a placebo. The results have not yet been reported.

In another phase I clinical trial of intravenous injection of allogeneic adipose tissue-derived MSCs for ARDS treatment (NCT01902082), 12 adult patients with ARDS were administered with a single intravenous injection of allogeneic adipose MSCs (1 million cells/kg), but the AT-MSCs treatment did not significantly reduce the levels of serum inflammatory cytokines (IL-6 and IL-8) in the ARDS patients, nor did it improve lung function in the ARDS patients, indicating that this was a safe but ineffective treatment.

Recent studies have found that extracellular vesicles (EVs) produced by MSCs promoted the phagocytic activity of alveolar macrophages, thereby reducing bacterial pneumonia induced by Gram-negative Escherichia coli [MonsclA, et al. Am J Respir Crit Care Med. 2015, 192(3): 324-336]. In addition, MSCs produce antimicrobial proteins that directly inhibit bacterial growth in inflamed lungs.

Intratracheal injection of MSCs significantly attenuated lung injury and inflammatory responses and improved survival in experimental animals with bacterial pneumonia by promoting bacterial clearance in a lipocalcin-2-dependent manner. LPS-induced activation of TLR-4 in MSCs enhanced the secretion of lipocalcin-2, which can bind to bacterial siderophores, reduce iron absorption, and inhibit bacterial growth. Consistent with these findings are recently reported findings: in an experimental model of bacterial pneumonia, Gupta et al. [Gupta N, et al. Thorax 2012, 67(6): 533-539] found mutations of TLR-4 in MSCs significantly diminished their therapeutic effect. Thus, a potential new cell-based therapy is expected that can potentially eliminate antibiotic-resistant Gram-negative strains from the lung.

The study of the present invention demonstrates significant advantages of extracellular vesicles of mesenchymal stem cells. Stem cells have great potential for the treatment of various diseases. The therapeutic effect of stem cells is largely dependent on their paracrine cytokines and mediated by extracellular vesicles (EVs) or exosomes.

EVs are nano-sized membrane-structured vesicles that mediate cell-to-cell communication. MSC-derived EVs contain cytokines, growth factors, signaling lipids, mRNAs, miRNAs/siRNAs and other substances.

More and more research evidences suggest that MSC-EVs may represent a new cell-free therapeutic approach with distinct advantages over existing MSCs therapies: 1) no risk of tumor formation in vivo; 2) lower immunogenicity; 3) the possibility of deriving EVs from endogenous MSCs and from the supernatant (of a conditioned medium) during the culture of allogeneic human MSCs through separation and purification, making it possible to meet the needs of clinical scale production and purification, with high yield in a batch and convenient batch quality control; 4) the possibility of in vitro modification of seed stem cells of specific proliferation passages producing EVs to concentrate more specific EVs for different therapeutic purposes; 5) while MSCs are easy to deposit and adhere to form micro-clots since the diameter of a single suspended MSC (18-40 µm) is 3-7 times bigger than the diameter of an erythrocyte (6 µm), leading to a risk of clogging of the capillaries after intravenous injection, EVs are nano-membrane structured vesicles with a diameter of 50-500 nm, which have good dispersibility in aqueous solution and are easy to prepare into an injection; 6) EVs have good membrane structure stability and strong tolerance of cryopreservation and freeze-thaw processes; 7) due to the membrane structure and nanoparticle characteristics, EVs can be actively phagocytosed and taken in by target tissue cells, thereby obtaining biological information and/or active ingredients carried by EVs to exert the direct signal transduction of EVs to cells and targeted drug delivery; 8) EVs can be loaded with certain active ingredients and become a natural drug delivery system; 9) a plurality of research groups have reported the therapeutic potential of MSC-derived EVs. MSCs-EVs have the potential as future biomarkers and therapeutic agents for ARDS. MSCs-EVs also appear to play a key role in ARDS recovery.

Multiple studies have found therapeutic advantages of MSC-derived microvesicles (MVs) via airway or intravenous routes. The study of Zhu et al. showed that intratracheal infusion of MSCs-derived MVs could reduce extracellular water content in lung tissue, reduce pulmonary edema, and reduce alveolar membrane permeability to proteins in E. coli endotoxin-induced lung injury. MSC-MVs also reduced neutrophil influx and reduced the level of macrophage inflammatory protein-2 in bronchoalveolar lavage fluid (BAL).

MSCs regulate macrophages in ALI through EVs-mediated mitochondrial transfer. Angiopoietin-1 is abundant in MSC-MVs, and the immunomodulatory properties of MSCs on macrophages are mediated in part by the transfer of angiopoietin-1 mRNA to macrophages. MSC exosomes partially improved pulmonary microvascular permeability in an LPS-induced acute lung injury model of mice by reducing endothelial cell apoptosis, increasing IL-10 production, and reducing IL-6 production through the hepatocyte growth factor (HGF).

MSCs-MVs can restore the integrity of tight junctions of alveolar membranes and can also reduce the permeability-enhancing effects of IL-1β, TNF-α and IFN-γ on human pulmonary microvascular endothelial cells. Pretreatment with anti-CD44 and angiopoietin-1 siRNA abolished this therapeutic effect of MSCs-MVs, suggesting that CD-44 and angiopoietin-1 mRNA transfer are involved in the repair and therapeutic mechanism of MSCs-MVs. The expression of MV subsets can be enriched by pre-treating MSCs, thereby increasing their therapeutic potential.

Based on the study of the inventors, the following inventions are provided:
**Invention 1:** A pharmaceutical formulation containing active ingredients derived from human cells, and the ingredients have good stability and high dispersibility, are easily absorbed by the human body other than the skin and are suitable for preventing and treating inflammation and injury caused by various reasons.
**Invention 2:** The active ingredients derived from human cells as described in invention 1, wherein they include but are not limited to extracellular vesicles and soluble cytokines produced by stem cells, progenitor cells and immune cells derived from human tissues, bone marrow and blood.
**Invention 3:** The pharmaceutical formulation as described in invention 1, including but not limited to an atomized inhalation liquid, eye drops, and nasal drops.
**Invention 4:** The pharmaceutical formulation as described in invention 1 has good stability, wherein the stability of the active ingredients is markedly better than preparations containing living cells under the same storage conditions, for example, without limited to, storage at room temperature, storage at a low temperature below -20°C, storage at a low temperature below - 70°C, storage at a low temperature below -135°C, and storage at a low temperature of -196°C.
**Invention 5:** The pharmaceutical formulation as described in invention 1 has high dispersibility, wherein it is colorless and transparent without visible floccules and precipitate when placed at 0-25°C for 6-24 hours.
**Invention 6:** The pharmaceutical formulation as described in invention 1 is easy for administration to sites of the human body other than the skin, wherein these sites include but are not limited to the bulbar conjunctiva, palpebral conjunctiva, retina, oral cavity, nasal cavity, upper respiratory tract, lower respiratory tract, gastrointestinal tract, vascular endothelial cells, type 1 alveolar epithelial cells, type 2 alveolar epithelial cells, mononuclear macrophages, neutrophils, dendritic cells, antigen presenting cells, T lymphocytes, fibroblasts, central nerve cells, and peripheral nerve cells and the fibers of their ending.
**Invention 7:** The pharmaceutical formulation as described in invention 1 is suitable for preventing and treating inflammation and injury caused by various factors, including but not limited to viral infectious inflammation, bacterial infectious inflammation, fungal infectious inflammation, autoimmune reactive inflammation, ischemic injury, chemical injury, and physical injury.
**Invention 8:** The stem cells, progenitor cells and immune cells derived from human tissues, bone marrow and blood as described in invention 2, including but not limited to mesenchymal stem cells derived from human adipose tissue, alveolar epithelial progenitor cells derived from human lung tissue, mesenchymal stem cells derived from Wharton's jelly of human umbilical cord, mesenchymal stem cells derived from human endometrial tissue, stem cells derived from human placental tissue, epithelial cells derived from human placental amniotic membrane, mononuclear cells in human blood, hematopoietic stem cells in human bone marrow tissue, mesenchymal stem cells in human bone marrow tissue, mesenchymal stem cells derived from human periosteum, keratinocytes derived from human skin tissue, dendritic cells derived from human blood tissue, CD4-positive T lymphocytes derived from human blood tissue, and platelets derived from human blood tissue.
**Invention 9:** The extracellular vesicles derived from human cells as described in invention 2, including but not limited to nanovesicles enveloped in a lipid bilayer membrane structure, with a diameter of 30-1,000 nm, containing different types of microribonucleic acids (miRNAs), small nuclear ribonucleic acid (sRNAs), non-coding DNA fragments, transfer ribonucleic acids (t-RNAs), soluble cytokines, growth factors, and other proteins of specific functions.
**Invention 10:** The soluble cytokines derived from human cells as described in invention 2, including but not limited to soluble and free lipoprotein molecules, glycoprotein molecules, biosignaling molecules, transforming growth factor β (TGF β), hepatocyte growth factor (HGF), vascular endothelial growth factor (VEGF), keratinocyte growth factor (KGF), interleukin 10 (IL-10), and interleukin 1β receptor antagonist (IL-1βA).
**Invention 11:** The soluble cytokines derived from human cells as described in invention 2 and invention 10, including but not limited to cytokines produced paracrinely from human somatic cells cultured in a natural state, and from overexpression of human somatic cells modified with specific genes.
**Invention 12:** The extracellular vesicles as described in invention 2 and invention 9, which specifically express membrane protein markers CD9, CD63, CD81 and TSG101, and do not express CANX. The diameter of the extracellular vesicles is preferably 50-500 nm.
**Invention 13:** The stem cells, progenitor cells, and immune cells derived from human tissues, bone marrow and blood as described in invention 2 and invention 8, wherein these cells may be obtained by direct separation from human tissues and purification, or by minimal manipulation in a GMP laboratory to proliferate the cells after direct separation from human tissues and purification, or by specific gene modification, specific gene editing, specific gene transduction, specific miRNA introduction in a GMP laboratory, or by pretreatment under special culture conditions in a GMP laboratory.
**Invention 14:** The stem cells, progenitor cells, and immune cells derived from human tissues, bone marrow and blood as described in invention 2 and invention 8, wherein these cells are preferably adipose-derived mesenchymal stem cells and placental amniotic membrane-derived mesenchymal stem cells.
**Invention 15:** The stem cells, progenitor cells, and immune cells derived from human tissues, bone marrow and blood as described in invention 2, invention 8, invention 13 and invention 14 are more preferably adipose-derived mesenchymal stem (progenitor) cells and placental amniotic membrane-derived mesenchymal stem cells.
**Invention 16:** The more preferably adipose-derived mesenchymal stem (progenitor) cells as described in invention 15, wherein fat is taken by abdominal liposuction or abdominal dermolipectomy from a healthy male or female volunteer who has passed ethical review, has been registered, has signed the informed consent, and has been strictly examined by a laboratory to meet the criteria for an adipose stem (progenitor) cell bank, placed in a patented cell storage solution, and transported at a low temperature to a qualified GMP laboratory, and adipose mesenchymal progenitor cells as working bank cells (intermediate product) are obtained by a patented separation, purification and proliferation process.
**Invention 17:** The more preferably human adipose tissue-derived mesenchymal progenitor cells as described in invention 15 and invention 16, wherein this specific type of human adipose tissue-derived mesenchymal stem (progenitor) cells are P1-P6 adipose mesenchymal stem (progenitor) cells, more preferably passage P3-P4 adipose mesenchymal stem (progenitor) cells, produced in accordance with a production process for intermediate products in a GMP production laboratory, tested negative for various pathogens, with a percentage of approximately 98% of specific surface markers such as CD73-positive, CD90-positive and CD105-positive cells and a percentage smaller than 2% of CD34-positive/CD45-positive and HLA-DR-positive cells, and meeting the detection criteria for antibiotic residues and serum and serum substitute residues.
**Invention 18:** The more preferably adipose tissue-derived mesenchymal stem (progenitor) cells as described in inventions 15 to 17 are stored under a deep hypothermic condition at -196°C to -80°C for 0-36 months, preferably, at -196°C to -135°C for 0-24 months.
**Invention 19:** The human somatic cell-derived extracellular vesicles and the soluble cytokines as described in invention 2 and inventions 9 to 12, wherein the production process thereof is:
   (1) Seeding these types of human somatic cells in an α MEM basal culture medium containing 5% EliteGro, culturing them in a HyperFlask culture flask under culture conditions of 37°C and 5% CO₂ until the cell confluency reaches 80%, then using a specific pretreated medium for culturing under culture conditions of 37°C and 5% CO₂ for 48 hours.
   (2) Collecting the cell culture supernatant and performing separation by differential centrifugation combined with polyethylene glycol (PEG) precipitation.
**Invention 20:** The differential centrifugation combined with polyethylene glycol (PEG) precipitation in (2) of the production process as described in invention 19, wherein, PEG of PEG 3000-PEG 9000 with PBS configured to 8%-30% is filtered with a 0.22 µm filter, added to a treated conditioned medium at a volume ratio of 1:1, placed at 4°C overnight for incubation, and centrifuged under 3,000-5,000 g at 4°C for 45-60 minutes, and the supernatant is removed. Pre-cooled PBS is added to resuspend the precipitate, ultracentrifugation is performed under 100,000-120,000 g at 4°C for 60-120 minutes, the supernatant is removed, an appropriate amount of isotonic sodium chloride solution is added, and the precipitate is resuspended to obtain the human somatic cell-derived extracellular vesicles and the soluble cytokines.
**Invention 21:** The extracellular vesicles obtained by the production process as described in invention 19 and invention 20, characterized in that they have biomarkers CD9 positive, CD63 positive, CD81 positive, TSG101 positive, and CANX negative.
**Invention 22:** The production as described in inventions 19 to 21, wherein, under the GMP laboratory conditions, it can be scaled up to a clinical production scale for cell products and has the following advantages: 1 production operation unit can separate 800-1,200 ml of conditioned medium from 2 cell factories to obtain 2-5 × 10¹¹ extracellular vesicles. One batch produced from one production operation unit can meet the need of 100-250 patients if a total of 2-5 × 10⁹ extracellular vesicles are used locally for each patient. One batch produced from 100 production operation units can yield the extracellular vesicles for use by 10,000-25,000 people for 5-6 days.
**Invention 23:** The production as described in inventions 19 to 22, wherein, the separated extracellular vesicles and the soluble cytokines are cross-linked with PEG of a specific molecular weight to form hydrophilic particles less than 3 microns which have high stability at a low temperature when stored frozen at -196°C to -20°C and have high dispersibility in an aqueous solution at room temperature, both superior to the prior art.
**Invention 24:** The extracellular vesicles, soluble cytokines and hydrophilic particles cross-linked by PEG as described in invention 23, which may be prepared into an atomized inhalation liquid, eye drops, nasal drops, or a topical gel preparation by dispersing them in a solution, including but not limited to, an isotonic sodium chloride solution, a low molecular hyaluronic acid solution, artificial tears or a gel solution. The intake of extracellular vesicles obtained by the process of the present invention by local mucosal cells is not affected by PEG.
**Invention 25:** The pharmaceutical formulation containing human somatic cell-derived extracellular vesicles and soluble cytokines as described in invention 1 and inventions 23 and 24 are preferably made into an atomized inhalation formulation suitable for the treatment of infectious lung injury.
**Invention 26:** The pharmaceutical formulation as described in invention 1 and invention 25, wherein it is preferably an atomized inhalation liquid prepared with extracellular vesicles and soluble cytokines of adipose tissue-derived mesenchymal stem (progenitor) cells and placental amniotic membrane-derived mesenchymal stem cells and an isotonic aqueous sodium chloride solution, and is suitable for atomized inhalation treatment of acute lung injury caused by infection of viruses, including but not limited to influenza viruses, SARS coronavirus, SARS-Cov-2, and MERS coronavirus, to prevent the release of acute lung injury inflammatory factors, reduce the infiltration of a high protein liquid in the alveoli and alveolar epithelium cell damage, significantly increase the success rate of rescue of patients with acute lung injury, and improve lung functions and the quality of life of surviving patients.

All documents mentioned in the present invention are cited as references in this application as if each document is individually cited. In addition, it should be understood that, after reading the above teaching of the present invention, those skilled in the art can make various changes or modifications to the present invention, and these equivalent forms also fall within the scope defined by the appended claims of this application.

## Claims

1. A pharmaceutical composition, **characterized in that** the pharmaceutical composition contains (a) an active ingredient derived from human somatic cells, which is extracellular vesicles produced by human somatic cells; and (b) a pharmaceutically acceptable carrier.

2. The pharmaceutical composition as described in claim 1, **characterized in that** the somatic cells are selected from the group consisting of: stem cells, progenitor cells or immune cells derived from human tissues, bone marrow and/or blood, or a combination thereof.

3. The pharmaceutical composition as described in either claim 1 or claim 2, **characterized in that** a dosage form of the pharmaceutical composition is selected from the group consisting of: an atomized inhalation preparation, eye drops, or nasal drops.

4. The pharmaceutical composition as described in any of claims 1 to 3, **characterized in that** the pharmaceutical composition has the following characteristics:
(P1) better storage stability than living cell formulations; preferably, the storage is at room temperature and/or a low temperature; "better storage stability than living cell formulations" means that the stability of the pharmaceutical composition is better than that of a formulation containing living cells under the same conditions; and
(P2) high dispersibility; preferably, when the pharmaceutical composition is a liquid formulation containing water (for example, a solution prepared with saline) and placed at 0-25°C for 6-24 hours, it is colorless and transparent without visible floccules or precipitation.

5. The pharmaceutical composition as described in any of claims 1 to 4, **characterized in that** the pharmaceutical composition is administered to a site selected from the group consisting of: the bulbar conjunctiva, palpebral conjunctiva, retina, oral cavity, nasal cavity, upper respiratory tract, lower respiratory tract, gastrointestinal tract, lung, or a combination thereof.

6. The pharmaceutical composition as described in any of claims 1 to 5, **characterized in that** the diameter of the extracellular vesicles is preferably 50-500 nm.

7. A method for preparing extracellular vesicles, **characterized by** comprising steps of:
(S1) culturing human somatic cells to a predetermined confluency (for example, 75-90%);
(S2) under conditions suitable for EV production, continuing the culture of the cells for a period of time T1;
(S3) removing the cells from the culture system to separate and obtain a culture medium containing extracellular vesicles, i.e., "conditioned medium";
(S4) mixing the conditioned medium with polyethylene glycol (PEG) to form a first mixture, and placing it for a period of time T2, thereby forming PEG-modified extracellular vesicles; T2 is usually 6-60 hours, preferably 12-48 hours;
(S5) centrifuging the first mixture from the previous step to precipitate the PEG-modified extracellular vesicles, and removing the supernatant to obtain a PEG-modified extracellular vesicle precipitate;
(S6) resuspending the PEG-modified extracellular vesicle precipitate obtained from the previous step to obtain a first resuspension mixture;
(S7) centrifuging the first resuspension mixture to precipitate the PEG-modified extracellular vesicles, and removing the supernatant to obtain a PEG-modified extracellular vesicle precipitate;
(S8) resuspending the PEG-modified extracellular vesicle precipitate obtained from the previous step to obtain a medically acceptable extracellular vesicle formulation;

8. An extracellular vesicle formulation, **characterized in that** the extracellular vesicle formulation is prepared by the method described in claim 7.

9. A use of the pharmaceutical composition as described in any of claims 1 to 6 or the extracellular vesicle formulation as described in claim 8, **characterized in that** they are used to prepare a drug for preventing and/or treating inflammation or injury.

10. The use as described in claim 9, **characterized in that** the inflammation is selected from the group consisting of: viral infectious inflammation, bacterial infectious inflammation, fungal infectious inflammation, autoimmune response inflammation, or a combination thereof;
the injury is selected from the group consisting of: ischemic injury, hypoxic injury, chemical injury, physical injury, or a combination thereof.

11. The use as described in claim 9, **characterized in that** the drug is used to treat an acute respiratory distress syndrome.

12. The use as described in claim 9, **characterized in that** the drug is an atomized inhalation liquid.

13. The use as described in claim 9, **characterized in that** the drug is used to treat viral acute lung injury.

14. The use as described in claim 13, **characterized in that** the drug is used for treatments through atomized inhalation to prevent releasing acute lung injury inflammatory factors and to alleviate infiltrating a high protein liquid in alveoli and an alveolar epithelium cell damage.
